# EUROPÄISCHE PATENTSCHRIFT

(11) **EP 0 290 862 B1**
(45) Veröffentlichungstag und Bekanntmachung des Hinweises auf die Patenterteilung: **23.06.1993**
(21) Anmeldenummer: 88106702.9
(22) Anmeldetag: 27.04.1988
(51) Int. Cl.: C07D 487/08

(54) **Verfahren zur Herstellung von 1,4-Diazabicyclo(2,2,2)-octanen**
Process for the preparation of 1,4-diazabicyclo[2.2.2] octanes
Procédé de préparations de 1,4-diazabicyclo[2.2.2]octanes

(30) Priorität: 12.05.1987 DE 3715753
(43) Veröffentlichungstag der Anmeldung: 17.11.1988
(73) Patentinhaber: BASF Aktiengesellschaft, 67063 Ludwigshafen (DE)
(72) Erfinder: Hoelderich, Wolfgang, Dr., D-6710 Frankenthal (DE); Schneider, Kurt, Dr., D-6702 Bad Duerkheim (DE); Ruge, Bernd, Dr., D-6737 Boehl-Iggelheim (DE)

(56) Entgegenhaltungen:
- DE-A- 2 434 913
- ULLMANNS ENCYCLOPAEDIE DER TECHNISCHEN CHEMIE, 4. Auflage, 1983, Band 24, Seiten 575-578, Verlag Chemie Weinheim; M. MENGEL: "Zeolithe"

## Beschreibung

Die vorliegende Erfindung betrifft ein Verfahren zur Herstellung von 1,4-Diazabicyclo(2,2,2)-octan (DABCO) und dessen C-substituierte Derivate durch Umsetzung heterocyclischer Amine der Formel (II) in Gegenwart von Aluminosilikatzeolithen als Katalysatoren, die alkalifrei synthetisiert worden sind.

Es ist bekannt, daß man DABCO und seine Derivate aus heterocyclischen Aminen heterogenkatalysiert herstellen kann. Als Heterogenkatalysatoren kamen bisher zum Einsatz Phosphate des Ca, Sr, Ba, Zn, La, Al, Co, Ni, Ce (EP 111 - 928, US 4 514 567, US 3 342 820, US 3 297 701, EP 69 322). Derartige gefällte Phosphate liefern unbefriedigende Ausbeuten.

Auch ist bekannt, daß man Aluminosilikatzeolithe des ZSM-Typs als Heterogenkatalysatoren für die Reaktion einsetzen kann (EP 158 319). Die Ausbeuten sind klein; bei niedrigem Umsatz werden hohe Selektivitäten, bei hohem Umsatz niedrige Selektivitäten erreicht. Bei Verwendung von A-Zeolithen (DE-PS 2 434 913) und Silica-Alumina-Crackkatalysatoren (US 3 166 558) als Katalysatoren werden ebenfalls nur niedrige Ausbeuten erzielt. Weiterhin weist das Reaktionsprodukt eine Reihe unerwünschter Nebenprodukte wie N-Alkylpiperazine und Pyrazin-Derivate auf. Die Nebenprodukte haben physikalische und chemische Eigenschaften ähnlich dem DABCO und können daher mit konventionellen physikalischen Methoden wie Destillation und Kristallisation nur schwer bzw. unvollständig abgetrennt werden. Für die Weiterverarbeitung werden an die Reinheit des DABCO hohe Anforderungen gestellt. Niedrige Ausbeuten erfordern daher eine aufwendige und kostspielige Reinigung.

Weiterhin sind neben den Phosphaten und Zeolithen eine Reihe Katalysatoren für die Herstellung von 1,4-Diazabicyclo(2,2,2)-octanen beschrieben, wie z.B. Kaolin (US 2 937 176), Al₂O₃ (DE 2 442 929), für die auch die oben erwähnten Nachteile gelten.

Es bestand daher die Aufgabe, Katalysatoren zu finden, die bessere Ausbeuten (Umsatz x Selektivität) an den gewünschten 1,4-Diazabicyclo(2,2,2)-octanen über längere Zeiten liefern und dadurch auch eine vereinfachte Reindarstellung des Endproduktes ermöglichen.

Es wurde gefunden, daß man bei der Herstellung von 1,4-Diazabicyclo(2,2,2)-octan und C-substituierte 1,4-Diazabicyclo(2,2,2)-octane der Formel (I) 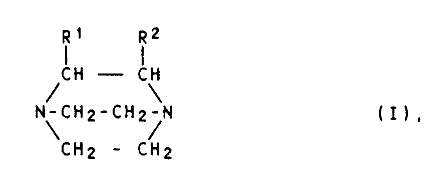
in der R₁ und R² Wasserstoff-, Alkyl- mit 1 - 4 C-Atomen oder Alkenylreste mit 1 - 4 C-Atomen bedeuten können überraschenderweise hohe Selektivitäten bei hohem Umsatz während langen Laufzeiten erzielt, wenn man heterocyclische Amine der Formel (II) 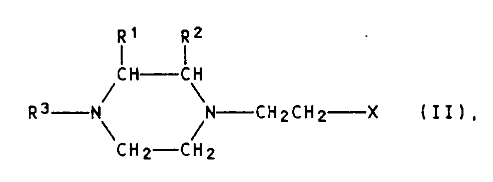
in der R¹ und R² obige Bedeutung haben und R³ Wasserstoff- und Hydroxyethyl- oder Aminoethylrest und X HO- oder H₂N- oder Hydroxyethyl- oder Aminoethylrest bedeuten, in Gegenwart von Aluminiumsilikatzeolithen als Katalysatoren umsetzt die ohne Zusatz von Alkali in wäßriger Hexamethylendiamin- oder Diaminpropan- oder Triethylentetraminlösung synthetisiert worden sind.

Als Ausgangsstoffe gemäß Formel II kann man z.B. Monohydroxyethylpiperazin, Dihydroxyethylpiperazin, Monoaminoethylpiperazin, Aminoethylhydroxyethylpiperazin verwenden. Es können auch Gemische von Verbindungen der Formel II verwendet werden, bevorzugt ist hierbei eine wäßrige Lösung aus Mono- und Dihydroxyethylpiperazin.

Zeolithe sind kristalline Aluminosilikate, die eine hochgeordnete Struktur mit einem starren dreidimensionalen Netzwerk von SiO₄- und AlO₄-Tetraedern besitzen, die durch gemeinsame Sauerstoffatome verbunden sind. Das Verhältnis der Si- und Al-Atome zu Sauerstoff beträgt 1 : 2 (siehe Ulmanns Encyclopädie d. techn. Chemie, 4. Auflage, Band 24, S. 575 (1983). Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekeln besetzt.

Die erfindungsgemäß verwendeten Aluminosilikatzeolithe werden z.B. bei 90°C bis 200°C unter autogenem Druck synthetisiert, indem man eine Aluminiumverbindung, z.B. Al(OH)₃, mit einer Siliciumverbindung, vorzugsweise hochdispersem Siliciumdioxid in wäßriger Aminlösung, insbesondere in 1,6-Hexandiamin-, 1,3-Propandiamin- oder Triethylentetraminlösung ohne Alkali- oder Erdalkalizusatz zur Reaktion bringt. Auch isotaktische Zeolithe nach DE-OS 3 006 471 und EP 46 504 können als Katalysatoren verwendet werden.

Die auf diese Weise hergestellten Aluminosilikatzeolithe kann man nach Isolierung, Trocknung bei 100 bis 160°C, vorzugsweise 110°C und Calcinierung bei 450 bis 550°C, vorzugsweise 500°C bis 540°C, mit einem Bindemittel im Verhältnis 90 : 10 bis 40 : 60 Gew.% zu Strängen oder Tabletten verformen. Als Bindemittel eignen sich verschiedene Aluminiumoxide, insbesondere Boehmit oder amorphe Aluminosilikate mit einem SiO₂/Al₂O₃-Verhältnis von 25 : 75 bis 95 : 5, insbesondere 75 : 25, Siliciumdioxid, vorzugsweise aber hochdisperses SiO₂, oder Gemische aus hochdispersem SiO₂ und hochdispersem Al₂O₃, sowie Ton. Nach der Verformung werden die Extrudate oder Preßlinge bei 110°C/16 h getrocknet und bei 500°C/16 h calciniert.

Man kann auch den isolierten Aluminosilikatzeolith direkt nach der Trocknung verformen und erstmals nach der Verformung der Calcinierung unterwerfen. Die Aluminosilikatzeolithe können in reiner Form, ohne Binder, als Stränge oder Tabletten eingesetzt werden, wobei als Verstrangungs- oder Peptisierungshilfsmittel, z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische, verwendet werden können.

Diese Aluminosilikatzeolithe liegen aufgrund ihrer Herstellung nach der Calcination sofort in der katalytisch aktiven, aciden H-Form vor, d.h. ein zeitaufwendiger Ionenaustausch z.B. mit Ammoniumionen und anschließende Calcinierung oder durch Behandlung mit Säuren kann unterbleiben.

Wenn bei der erfindungsgemäßen Verwendung der zeolithischen Katalysatoren durch Koksabscheidung eine Deaktivierung eintritt, empfiehlt es sich, die Zeolithe durch Abbrennen der Koksablagerung mit Luft oder mit einem Luft/N₂-Gemisch bei 400 bis 500°C, vorzugsweise 500°C bis 540°C, zu regenerieren. Die Zeolithe erhalten dadurch ihre Anfangsaktivität zurück. Durch partielle Verkokung (pre-coke) ist es möglich, die Aktivität des Katalysators für ein Selektivitätsoptimum des gewünschten Reaktionsproduktes einzustellen.

Um eine möglichst große Selektivität, hohen Umsatz sowie lange Standzeiten zu erreichen, ist es mitunter vorteilhaft, die Zeolithe zu modifizieren. Eine geeignete Modifizierung der Katalysatoren besteht z.B. darin, daß man die unverformten oder verformten Zeolithe mit Phosphorverbindungen oder Metallsalzen durch einen Ionenaustausch oder durch Imprägnierung dotiert. Als Metalle werden Metalle der 2., 3., 4. und 5. Hauptgruppe wie Mg, Ca, Sr, Al, Ga, Ge, Sn, Pb, Bi, Übergangsmetalle der 4. - 8. Nebengruppe wie Ti, Zr, V, Nb, Cr, Mo, W, Mn, Re, Fe, Ru, Os, Co, Rh, Ir, Ni, Pd, Pt, Übergangsmetalle der 1. und 2. Nebengruppe wie Cu, Ag, Zn, seltene Erdmetalle wie La, Ce, Pr, Nd, Er, Yb und U eingesetzt.

Zweckmäßigerweise führt man die Dotierung so durch, daß man z.B. die verformten Zeolithe in einem Steigrohr vorlegt und bei 20 bis 100°C z.B. eine wäßrige oder ammoniakalische Lösung eines Halogenids oder eines Nitrats der Metalle überleitet. Ein derartiger Ionenaustausch kann z.B. an der Wasserstoff-, Ammonium- und Alkaliform der Zeolithe vorgenommen werden. Eine weitere Möglichkeit der Metallaufbringung auf Zeolithe besteht darin, daß man das zeolithische Material z.B. mit einem Halogenid, einem Nitrat oder einem Oxid der Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine zweckmäßige Ausführungsform besteht darin, daß man Cu(NO₃)₂ der Ni(NO₃)₂, Ce(NO₃)₃, La(NO₃)₃ oder Cs₂CO₃ in Wasser löst und mit der Lösung die verformte oder unverformten Zeolithe eine gewisse Zeit, etwa 30 Minuten, tränkt. Die gegebenenfalls überstehende Lösung wird am Rotationsverdampfer von Wasser befreit. Danach werden die getränkten Zeolithe bei etwa 150°C getrocknet und bei etwa 550°C calciniert. Dieser Tränkvorgang kann mehrmals hintereinander vorgenommen werden, um den gewünschten Metallgehalt einzustellen.

Es ist auch möglich, eine wäßrige Ni(NO₃)₂-Lösung oder eine ammoniakalische Pd(NO₃)₂-Lösung herzustellen und darin die reinen pulverförmigen Zeolithe bei 40 bis 100°C unter Rühren etwa 24 h aufzuschlämmen. Nach Abfiltrieren, Trocknen bei etwa 150°C und Calcinierung bei etwa 500°C kann das so gewonnene zeolithische Material mit oder ohne Bindemittel zu Strängen, Pellets oder Wirbelgut weiterverarbeitet werden.

Ein Ionenaustausch an den in der H-Form vorliegenden Zeolithen kann so vorgenommen werden, daß man die Zeolithe in Strängen oder Pellets in einer Kolonne vorlegt und darüber eine wäßrige Ni(NO₃)2-Lösung oder eine ammoniakalische Pd(NO₃)₂-Lösung bei leicht erhöhter Temperatur zwischen 30 und 80°C im Kreislauf 15 bis 20 h leitet. Danach wird mit Wasser ausgewaschen, bei etwa 150°C getrocknet und bei etwa 550°C calciniert.

Bei manchen metalldotierten Zeolithen, z.B. Pd-, Cu-dotierten Zeolithen, ist eine Nachbehandlung mit Wasserstoff vorteilhaft.

Eine weitere Möglichkeit der Modifizierung besteht darin, daß man das zeolithische Material- verformt oder unverformt - einer Behandlung mit Säuren wie Salzsäure, Flußsäure und Phosphorsäure und/oder Wasserdampf unterwirft.

Dabei geht man vorteilhaft so vor, daß man die Zeolithe in Pulverform mit 1 n Phosphorsäure 1 Stunde bei 80°C behandelt. Nach der Behandlung wird mit Wasser gewaschen, bei 110°C/16 Stunden getrocknet und bei 500°C/20 Stunden calciniert. Nach einer anderen Arbeitsweise behandelt man Zeolithe vor oder nach ihrer Verformung mit Bindemitteln, z.B. 1 bis 3 Stunden bei Temperaturen von 60 bis 80°C mit einer 3 bis 25 gew.%igen, insbesondere 12 bis 20 gew.%igen wäßrigen Salzsäure. Anschließend werden die so behandelten Zeolithe mit Wasser gewaschen, getrocknet und bei 400°C bis 500°C calciniert.

Eine besondere Ausführungsform für die Säurebehandlung besteht darin, daß man das zeolithische Material vor der Verformung bei erhöhter Temperatur mit Flußsäure, im allgemeinen mit 0,001 n bis 2 n, vorzugsweise 0,05 n Flußsäure behandelt, beispielsweise durch Erhitzen unter Rückfluß über einen Zeitraum von 0,5 bis 5, vorzugsweise 1 bis 3 Stunden. Nach Isolierung, z.B. durch Abfiltrieren und Auswaschen, des zeolithischen Materials wird dieses zweckmäßig, bei Temperaturen von 100 bis 160°C, getrocknet und bei Temperaturen von 450°C bis 600°C calciniert. Nach einer bevorzugten Ausführungsform für die Säurebehandlung wird das zeolithische Material nach einer Verformung mit Bindemittel bei erhöhter Temperatur, zweckmäßig bei 50°C bis 90°C , insbesondere 60°C bis 80°C, über einen Zeitraum von 0,5 bis 5, mit 12 bis 20 gew.%iger Salzsäure, behandelt. Anschließend wird das zeolithische Material ausgewaschen und z.B. bei Temperaturen von 100 bis 160°C, getrocknet und bei 450 bis 600°C calciniert. Einer HF-Behandlung kann sich auch eine HCl-Behandlung anschließen.

Die hier beschriebenen Katalysatoren können wahlweise als 2 bis 4 mm Stränge oder als Tabletten mit 3 bis 5 mm Durchmesser oder als Pulver mit Teilchengrößen von 0,1 bis 0,5 mm oder als Wirbelkontakt eingesetzt werden.

Die erfindungsgemäße Umwandlung wird in der Regel bevorzugt in der Gasphase bei 200 bis 550°C , insbesondere 300 bis 450°C, und einer Belastung WHSV = 0,1 bis 20 h⁻¹, insbesondere 0,2 bis 5 h⁻¹ (Ausgangsstoff/g Katalysator und Stunde) ausgeführt. Die Reaktion kann im Festbett oder Wirbelbett durchgeführt werden. Im allgemeinen steigt der Umsatz mit steigender Temperatur stark an, während die Selektivität in einem bestimmten Temperaturbereich nur wenig zurückgeht.

Es ist auch möglich, die Reaktion in der Flüssigphase (Suspension-, Riesel- oder Sumpffahrweise) durchzuführen.

Das Verfahren kann bei Normaldruck, erhöhtem oder vermindertem Druck, und diskontinuierlich oder vorzugsweise kontinuierlich durchgeführt werden.

Schwerflüchtige oder feste Ausgangsstoffe können in gelöster Form z.B. in wäßriger THF-, Toluol- oder Petrolether-Lösung zum Einsatz gebracht werden. Auch eine Verdünnung mit Lösungsmitteln wie den voranstehend genannten oder mit Inertgasen wie Stickstoff und Argon ist möglich.

Nach der Umsetzung werden die entstandenen 1,4-Diazabicyclo(2,2,2)-octane nach üblichen Techniken, z.B. durch Destillation aus dem Reaktionsgemisch isoliert; nichtumgesetzte Ausgangsstoffe werden gegebenenfalls, in die Umsetzung zurückgeführt.

Die in den Beispielen für das erfindungsgemäße Verfahren verwendeten Katalysatoren sind:

### Katalysator A

Der Aluminosilikatzeolith vom Pentasiltyp wird unter hydrothermalen Bedingungen bei autogenem Druck und 150°C aus 650 g hochdispersem SiO₂, 203 g Al₂(SO₄)₃ x 18 H₂O in 10 kg einer wäßrigen 1,6-Hexandiamin-Lösung (Mischung 50 : 50 Gew.% ) in einem Rührautoklaven hergestellt. Nach Abfiltrieren und Auswaschen wird das kristalline Reaktionsprodukt bei 110°C/24 h getrocknet und bei 500°C/24 h calciniert. Der Aluminosilikatzeolith enthält 92,8 Gew.% SiO₂ und 4,2 Gew.% Al₂O₃.

Katalysator A wird enhalten, indem man den reinen Aluminosilikatzeolithen des Pentasiltyps mit Boehmit im Gewichtsverhältnis 60 : 40 zu 2-mm-Strängen verformt, bei 110°C/16 h trocknet und bei 500°C/24 h calciniert.

### Katalysator B (Vergleichskatalysator)

Ein Aluminosilikatzeolith als Katalysator B wird nach US-PS 3 702 886, Beispiel 1, synthetisiert. Dieser Zeolith wird mit Boehmit im Gewichtsverhältnis 60 : 40 verformt, bei 110°C getrocknet und bei 500°C/16 h calciniert. Diese Stränge werden mit 20 %iger NH₄Cl-Lösung bei 80°C/2 h nach üblicher Methode ionenausgetauscht bis der Na-Gehalt 0,02 Gew.% (nach Trocknung bei 110°C und Calcination bei 500°C/5 h) liegt.

In den nachfolgenden Beispielen sind die mit obigen Katalysatoren erzielten Versuchsergebnisse und Versuchsbedingungen aufgezeigt.

### Einsatzlösung I:

Gemisch aus Mono- und Dihydroxyethylpiperazin (Gewichtsverhältnis 22 : 78 g) in Wasser gelöst 50 g : 50 g.

### Beispiel I

Auf den Kopf eines elektrisch geheizten Glasofens (Durchmesser 55 mm), dessen oberer Teil zur Verdampfung der Einsatzlösung mit Porzellanringen gefüllt ist und in dessen unterem Teil sich 200 g des Katalysators A befinden, läßt man 100 g der Einsatzlösung zulaufen. Die Temperatur im Ofen wird bei 345 bis 350°C und der Druck bei 400 mbar gehalten. Das aus dem Glasofen austretende Reaktionsgemisch wird in einem mit einem Rückflußkühler versehenen Glaskolben und in zwei nachgeschalteten Tiefkühlfallen aufgefangen und in bekannter Weise aufgearbeitet. Über 200 h Reaktionszeit wird ein durchschnittlicher Selektivitätswert von 86 % bei vollständigem Umsatz erzielt.

### Beispiel 2 (Vergleichsbeispiel)

Im Beispiel 2 wird wie bei Beispiel 1 verfahren, jedoch wird hierbei Katalysator B eingesetzt. Die Selektivität beträgt nunmehr 76 % bei vollständigem Umsetz. Bereits nach 95 h tritt bei Katalysator B Desaktivierung ein.

## Patentansprüche

1. Verfahren zur Herstellung von 1,4-Diazabicyclo(2,2,2)-octan und C-substituierten 1,4-Diazabicyclo(2,2,2)-octanen der Formel (I) 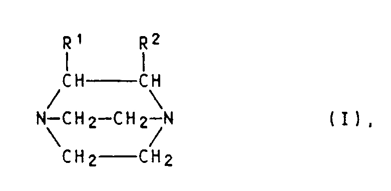 in der R¹ und R² Wasserstoff-, Alkyl- mit 1 - 4 C-Atomen oder Alkenylreste mit 1 - 4 C-Atomen bedeuten können, dadurch gekennzeichnet. daß man heterocyclische Amine der Formel (II) 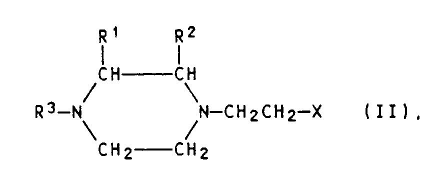 in der R¹ und R² obige Bedeutung haben und R³ Wasserstoff- oder Hydroxyethyl- oder Aminoethylrest und X HO- oder H₂N- oder Hydroxyethyl- oder Aminoethylrest bedeuten, in Gegenwart von Aluminosilikatzeolithen als Katalysatoren umsetzt, die ohne Zusatz von Alkali in wäßriger Hexamethylendiamin- oder Diaminopropan- oder Triethylentetraminlösung synthetisiert worden sind.

2. Verfahren nach Anspruch 1, dadurch gekennzeichnet, daß man Monohydroxyethylpiperazin oder Dihydroxyethylpiperazin oder deren Gemische als Ausgangsstoffe verwendet.

3. Verfahren nach den Ansprüchen 1 und 2, dadurch gekennzeichnet, daß man als Katalysatoren Aluminosilikatzeolithe des Pentasiltyps verwendet.

4. Verfahren nach Anspruch 1, dadurch gekennzeichnet, daß man mit Seltenen Erden modifizierte Aluminosilikatzeolithe als Katalysatoren verwendet.

5. Verfahren nach den Ansprüchen 1 bis 3, dadurch gekennzeichnet, daß man mit Übergangsmetallen modifizierte Aluminosilikatzeolithe als Katalysatoren verwendet.

6. Verfahren nach Anspruch 1, dadurch gekennzeichnet, daß man mit Phosphorverbindungen modifizierte Aluminosilikatzeolithe als Katalysatoren verwendet.

7. Verfahren nach den Ansprüchen 1 bis 6, dadurch gekennzeichnet, daß man die Reaktion in der Gasphase durchführt.

## Claims

1. A process for the preparation of 1,4-dizabicyclo[2.2.2]octane or a C-substituted 1,4-diazabicyclo[2.2.2]-octane of the formula (I) 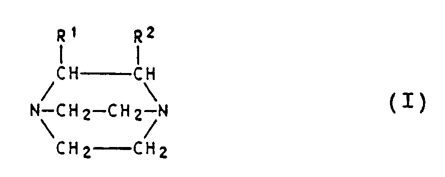 where R¹ and R² are each hydrogen or an alkyl or alkenyl radical of 1 to 4 carbon atoms, wherein a heterocyclic amine of the formula (II) 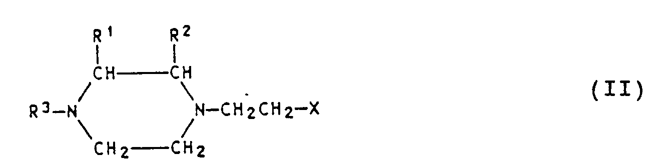 where R¹ and R² have the above meanings and R³ is hydrogen, hydroxyethyl or aminoethyl and X is HO- or H₂N-or hydroxyethyl or aminoethyl, is converted in the presence of an aluminosilicate zeolite as a catalyst, which has been prepared without the addition of an alkali in aqueous hexamethylenediamine or diaminopropane or triethylenetetramine solution.

2. A process as claimed in claim 1, wherein monohydroxyethylpiperazine or dihydroxyethylpiperazine or a mixture of these is used as a starting material.

3. A process as claimed in claim 1 or 2, wherein an aluminosilicate zeolite of the pentasil type is used as the catalyst.

4. A process as claimed in claim 1, wherein an aluminosilicate zeolite modified with rate earth metals is used as the catalyst.

5. A process as claimed in any of claims 1 to 3, wherein an aluminosilicate zeolite modified with transition metals is used as the catalyst.

6. A process as claimed in claim 1, wherein an aluminosilicate zeolite modified with phosphorus compounds is used as the catalyst.

7. A process as claimed in any of claims 1 to 6, wherein the reaction is carried out in the gas phase.

## Revendications

1. Procédé de préparation de 1,4-diazabicyclo[2.2.2]-octane et de 1,4-diazabicyclo[2.2.2]-octanes C-substitués de formule (I) 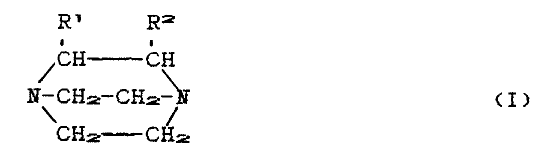 dans laquelle R¹ et R² peuvent représenter des atomes d'hydrogène, des restes alkyle à 1-4 atomes de carbone ou alcényle à 1-4 atomes de carbone, caractérisé en ce qu'on fait réagir des amines hétérocycliques de formule (II) 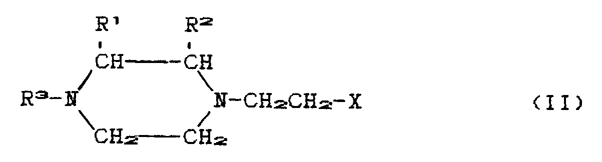 dans laquelle R¹ et R² ont les significations susindiquées et R³ représente un atone d'hydrogène ou un reste hydroxyéthyle ou aminoéthyle et X représente un reste HO- ou H₂N- ou un reste hydroxyéthyle ou aminoéthyle, en présence de zéolithes d'aluminosilicate qui servent de catalyseur et qui ont été synthétisées, sans addition de substance alcaline, en solution aqueuse d'hexaméthylènediamine, de diaminopropane ou de triéthylènetétramine.

2. Procédé selon la revendication 1, caractérisé en ce qu'on utilise, comme substances de départ, de la monohydroxyéthylpipérazine, de la dihydroxyéthylpipérazine ou des mélanges de celles-ci.

3. Procédé selon la revendication 1 ou 2, caractérisé en ce qu'on utilise, comme catalyseurs, des zéolithes d'aluminosilicate du type pentasil.

4. Procédé selon la revendication 1, caractérisé en ce qu'on utilise, comme catalyseurs, des zéolithes d'aluminosilicate modifiées par des terres rares.

5. Procédé selon l'une quelconque des revendications 1 à 3, caractérisé en ce qu'on utilise, comme catalyseurs, des zéolithes d'aluminosilicate modifiées par des métaux de transition.

6. Procédé selon la revendication 1, caractérisé en ce qu'on utilise, comme catalyseurs, des zéolithes d'aluminosilicate modifiées par des composés du phosphore.

7. Procédé selon l'une quelconque des revendications 1 à 6, caractérisé en ce qu'on conduit la réaction en phase gazeuse.
